# EUROPEAN PATENT APPLICATION

(11) **EP 1 271 385 A2**
(43) Date of publication of application: **02.01.2003**
(21) Application number: 02013824.4
(22) Date of filing: 21.06.2002
(51) Int. Cl.: G06F 19/00

(54) **System for and method of aiding to confirm medical care item, and computer program**

(30) Priority: 21.06.2001 JP 2001188810
(71) Applicant: Kameda, Toshitada, Kamogawa-shi, Chiba-ken (JP)
(72) Inventor: Kameda, Toshitada, Kamogawa-shi, Chiba-ken (JP); Hayashi, Akio, 3-18-16 Toranomon, Minato-ku, Tokyo (JP)
(74) Representative: Viering, Jentschura & Partner

(57) **Abstract**

A system (1) for aiding to confirm a medical care item is provided with: a search device (42) for searching a database (21) storing medical care data with a search condition corresponding to a plurality of medical care items and a plurality of patients to be confirmed, the medical care data including (i) medical care item data indicating the medical care item in one condition of a plurality of types of conditions, which are set in advance, (ii) condition data indicating the one condition, and (iii) patient identification data indicating the patient associated with an execution of the medical care item; a display device (41, 5) for displaying a table in a matrix shape in which the patient is set on a first axis and the medical care item is set on a second axis on the basis of the medical care data which is obtained as a search result by the search device and for displaying one symbol mark assigned to one condition indicated by the condition data included in the medical care data obtained as the search result, among a plurality of symbol marks assigned to a plurality of types of condition marks respectively, within a corresponding cell on the table; and an input device (3) for designating a desired cell or symbol mark, or a desired patient on the displayed table.

## Description

### BACKGROUND OF THE INVENTION

### 1. Field of the Invention

The present invention relates to a new system for and a new method of aiding or navigating a person related to medical care such as a medical doctor, a nurse and so on, to confirm various types of medical care items such as a prescription, an injection, an examination etc., which may be performed by a nurse or the like to a plurality of patients, according to instructions given by a doctor or the like at a health care facility such as a hospital or the like, for example. The present invention also relates to a computer program, which allows a computer to function as the aiding system.

### 2. Description of the Related Art

Conventionally, at a health care facility such as a hospital or the like, a plurality of nurses or the like generally make some groups, and each of the groups take charge of many patients, for example, by a unit of a hospital ward, by a unit of a hospital sector, or by a unit of a hospital department. Consequently, at a health care facility, a task table called "cardex" or the like is made by a unit of the hospital ward or the like, and the health care facility adopts such a mechanism that a plurality of nurses use the table and confirm various types of medical care items instructed by one or a plurality of doctors.

Moreover, recent advances of a computer have developed a system for confirming the various types of medical care items. As one example, there is a computer program called "a navigation care map" or "a care map" (both of which are registered trade marks of Kameda medical care information laboratory in Japan). By executing the computer program on a computer, the so-called "care map" is displayed on a picture plane of the computer. Here, the care map is a table in which a date is set on an abscissa, i.e. a horizontal axis, and a medical care item is set on an ordinate, i.e. a vertical axis. It is possible for a nurse or the like to confirm a schedule for each patient by using the displayed care map. Incidentally, this kind of care map is disclosed in Japanese Patent No. 2706645 (Japanese Patent Application Laying Open NO. Hei 9-185651) corresponding to U.S.P. 5,913,197 and Japanese Patent No. 2815346 (Japanese Patent Application Laying Open NO. Hei 10-214302) corresponding to U.S.P. 5,923,018, which have been applied by the present inventor.

However, even if various types of the above mentioned conventional task tables are displayed on a picture plane or a paper surface, under such a mechanism that a plurality of nurses perform various types of the medical care items to a plurality of patients, it is not certain on the display whether the nurses confirm an order issued or given by a doctor or the like, and further whether the nurses actually execute it after the order is displayed on the picture plane or the paper surface. This can cause a partial lack of confirmation from the schedule, a partial lack of execution and the like.

The use of the above mentioned care map enables the nurse or the like to confirm the schedule easily and certainly by a unit of the patient. However, the confirming becomes complicate and troublesome as objective patients increase in number if a nurse or the like uses the table in order to confirm the schedules for a plurality of or many patients, because the care map is a table made for a unit of the patient and because each of the care maps is different from each other, which causes a lack of practical use. Furthermore, there is the possibility that a certain nurse becomes extremely busy or has too much free time on a certain day because the display of the care map does not enable a nurse or the like who takes charge of a plurality of patients to easily understand the amount of the whole tasks and a task order such as what to be done next or what to be done on the day etc.

In addition, in the case that an administration period of medicines or injections which has been used continuously as a result of the instruction given by a doctor or the like is close to an end, it is extremely difficult for a nurse or the like who handles patients, or even for the doctor himself to confirm whether or not they should further continue the administration certainly or without any excess and deficiency, which causes a nurse or the like stressful. Actual occurrence of an expired prescription may give harmful effect to the medical care.

### SUMMARY OF THE INVENTION

It is therefore an object of the present invention to provide a system for and a method of aiding to confirm a medical care item, which enables confirming of a plurality of medical care items so as to certainly execute the medical care items scheduled for a plurality of patients, as well as a computer program, which allows a computer to function as the aiding system.

The above object of the present invention can be achieved by a system for aiding to confirm a medical care item. The system is provided with: a search device for searching a database storing medical care data with a search condition corresponding to a plurality of medical care items and a plurality of patients to be confirmed, the medical care data including (i) medical care item data indicating the medical care item in one condition (i.e., one status) of a plurality of types of conditions (i.e., a plurality of types of statuses), which are set in advance, (ii) condition data indicating the one condition, and (iii) patient identification data indicating the patient associated with an execution of the medical care item; a display device for displaying a table in a matrix shape in which the patient is set on a first axis and the medical care item is set on a second axis on the basis of the medical care data which is obtained as a search result by the search device and for displaying one symbol mark assigned to one condition indicated by the condition data included in the medical care data obtained as the search result, among a plurality of symbol marks assigned to a plurality of types of condition marks respectively, within a corresponding cell on the table; and an input device for designating a desired cell or symbol mark, or a desired patient on the displayed table, the display device displaying at least one portion of the cell or the symbol mark designated by the input device or the medical care data associated with the patient in a predetermined format different from a format of the table in place of or overlapped with the table, or together with the table, the input device being constructed to change the condition data indicated by the symbol mark displayed by the display device, the display device displaying the symbol mark indicating the condition data which is changed within the corresponding cell after the input device changing the condition data.

According to the aiding system of the present invention, the database constructed in the aiding system or connected to the aiding system through the communication device stores the medical care data including (i) the medical care item data indicating the medical care item in one condition among a plurality of types of conditions set in advance (e.g., the condition that the medical care item is scheduled by the doctor or the like, the condition that the order associated with the medical care item has been issued or given by the doctor or the like, the condition that the order associated with the medical care item has been confirmed by the nurse or the like, the condition that the medical care item has been executed by the nurse or the like, etc.), (ii) the condition data indicating the one condition, and (iii) the patient identification data indicating the patient associated with the execution of the medical care item. On the other hand, a plurality of the symbol marks are assigned to the plurality of types of conditions respectively. For example, a symbol mark showing "○" is assigned to the condition that the order has been issued or given by the doctor or the like. A symbol mark showing "―" is assigned to the condition that the order associated with the medical care item has been confirmed by the nurse or the like. A symbol mark showing "Δ" is assigned to the condition that the medical care item is scheduled by the doctor or the like. A symbol mark showing "×" is assigned to the condition that the medical care item has been executed by the nurse or the like. Incidentally, those kinds of symbol marks include a mark in an arbitrary picture pattern, a mark in colors or in black and white, a short text-sentence, alphabets, Japanese word, a blank (empty), and so on.

During the operation, the search device searches the database with the search condition in correspondence with the plurality of the patients to be confirmed (e.g., the plurality of the patients for the same hospital ward, for the same department, for the same doctor who takes charge of the patients, for the same group of the nurses who take charge of the patients etc.) and the plurality of the medical care items (e.g., the medical care item such as a prescription, an injection, an examination etc.). In addition, the search device searches the database with such a search condition that an execution date, an order issued date etc. associated with each medical care is included within designated days on the basis of the present date as a standard (e.g., in four days or in two days from the present date or from a standard day). Alternatively, as the search condition, the whole patients, the whole medical care items, the whole period etc. can be accepted, and the search condition such as only one patient, only one medical care item, only one day (the present date) etc. can be also accepted.

Next, the display device displays a table in a matrix shape in which the patient is set on the first axis (e.g. an ordinate or a vertical axis) and the medical care item is set on the second axis (e.g. an abscissa or a horizontal axis) on the basis of the medical care data which is obtained as a search result, and also displays the symbol mark assigned to the condition indicated by the condition data included in the medical care data obtained as the search result within the corresponding cell on the table. Next, when the desired cell or symbol mark or the desired patient on the displayed table is designated by the input device, the display device displays at least one portion of the medical care item associated with the cell or the symbol mark or the patient designated in the manner in a predetermined format. For example, the display device text-displays the medical care data indicating the details about the patient and the medical care item corresponding to the cell where the designated symbol mark is positioned, and the display device further text-displays the medical data by a detailed item. Alternatively, the display device may displays various types of task tables or work sheets relating to the designated patient and the designated medical care item. After the condition data indicated by the symbol mark displayed by the display device are changed by using the input device, the display device displays the symbol mark indicating the condition data which is changed within the corresponding cell.

Therefore, it is possible for the nurse or the like to take a survey of the conditions of the plurality of the medical care items relating to the plurality of patients (e.g., the plurality of the patients whom the nurse herself or the group to which she belongs handle), by looking at the table having the symbol mark indicating the condition of the medical care item, such as the item scheduled by the doctor or the like and the item for which the order is issued by the doctor or the like, within the corresponding cell thereof. This makes it possible to decrease the possibility of occurring a confirmation error and malpractice. More concretely, in the case that the nurse or the like has not confirmed an order, although the doctor or the like issued or gave the order, the symbol mark indicating the condition before the confirmation is kept displaying, so that it is possible to decrease the possibility that the nurse or the like overlooks it.

In one aspect of the system of the present invention, the input device is constructed to change the condition data corresponding to the medical care data displayed in the predetermined format by the display device..

According to this aspect, the condition data corresponding to the medical care data displayed in the predetermined format are changed by designating the desired cell or symbol mark or the desired patient on the table by the input device. For example, after the medical care data text-displayed is confirmed, the condition data corresponding to the medical care data are updated from the one indicating the unconfirmed condition (i.e., the unconfirmed status) to the another indicating the confirmed condition (i.e., the confirmed status) by the predetermined operation such as mouse-clicking, key-stroking, and so on. Consequently, the symbol mark corresponding to the updated condition data is displayed within the corresponding cell on the table. Namely, it is possible to perform the confirming of each medical care item though the display of the table and the display in the predetermined format.

In this aspect, the medical care item may be subdivided into detailed items, and the condition data corresponding to one cell within the table may comprise a plurality of detailed condition data indicating a condition of each one of the detailed items, and the display device may display at least one portion of the medical care data for each of the detailed items as the predetermined format, and the input device may be constructed to change the detailed condition data corresponding to the medical care data displayed in the predetermined format by the display device, and to change the condition data including the detailed condition data by changing the detailed condition data.

By constituting the aiding system in this manner, the condition data including the detailed condition data is changed by the input device by changing the detailed condition data corresponding to the medical care data displayed as the predetermined format for each of the detailed items. For example, if the condition data as for the whole detailed items constituting one medical care item are updated to the one indicating that the order has been confirmed, the condition data as for the one medical care item including these detailed items are also updated to the one indicating that the order has been confirmed.

In this case, moreover, the input device may be constructed to designate a desired portion from among the medical care data displayed for each of the detailed items in the predetermined format, and the display device may display the medical care data associated with the desired portion designated by the input device, in another format.

By constituting the system in this manner, if the desired portion from among the medical care data displayed for each of the detailed items in the format different from that of the table is designated by the input device, the medical care data associated with the desired portion is displayed in another different format. Thus, it is possible to confirm the content of the medical care data hierarchically or step-by-step.

In another aspect of the aiding system, the plurality of conditions include at least one of a condition that the medical care item is being scheduled for execution, a condition that an order associated with the medical care item has been issued or given, a condition that the order associated with the medical dare item has been confirmed, and a condition that the medical care item has been executed.

By constituting the aiding system in this manner, it is possible for the nurse, the doctor or the like to understand visually and easily in which step each medical care item such as the prescription, the injection, the examination etc., as for the plurality of the patients is e.g., in the step that the medical care item is just being scheduled, in the step that the order is issued or given, in the step that the order is confirmed, or in the step that the order is executed, by looking at the table, which is extremely useful in practice.

In another aspect of the system of the present invention, the display device displays the medical care data on the basis of the condition data in a different mode in correspondence with the condition thereof as the predetermined format.

According to this aspect, the display device displays the medical care data in a different mode in correspondence with the condition thereof, in the format different from that of the table. For example, under the condition that the order is issued or given, the display device text-displays the medical care data in red, and under the condition that the order is confirmed, it text-displays the medical care data in black. Consequently, it is possible for the nurse, the doctor or the like to understand the condition data on the medical care data displayed in the format different from that of the table, easily and visually.

Incidentally, the aiding system may be constructed such that the corresponding condition data is changed by an operation such as mouse-clicking etc. for the medical care data displayed in the format different from that of the table. By constituting the aiding system in this manner, the display mode changes from red to black by the changing of the condition data, for example, so that it is possible to lower the possibility that that error relating to the changing or the confirming occurs.

In another aspect of the aiding system of the present invention, the display device displays an examination result in another format in correspondence with the designation by the input device as for the medical care item which is displayed in the predetermined format and is accompanied with the examination result.

According to this aspect, as for the medical care item displayed in the predetermined format and is accompanied with the examination result, (e.g., an executed examination), the display device displays the examination result data in another format in correspondence with the designation by the input device. Because it is possible to display the examination result data through the display by the table to the display in the predetermined format, the system is extremely useful in practice.

In another aspect of the system of the present invention, the database updates a content stored therein in correspondence with a change of the condition data by the input device, and the database stores at least one of (i) operator identification data identifying an operator who executes the change of the condition data by the input device and (ii) change date data indicating a date when the change of the condition data by the input device is executed, with correlating the one with the condition data.

According to this aspect, the database is updated when the condition data are changed. At this occasion, the operator identification data indicating the operator (e.g. a certain nurse etc.) who executes the changing and the change date data indicating the date when the changing is executed are also stored in the form correlated with the condition data, so that it is possible to specify or confirm the operator and/or the date associated with the changing later.

In another aspect of the system of the present invention, the display device displays at least one of the operator identification data and the change date data in addition to or in place of the table.

According to this aspect, the operator identification data and/or the change date data displayed in addition to or in place of the table are displayed. Thus, by looking at them, it is possible to confirm the operator (e.g. the certain nurse etc.) who executed the changing of the condition data and/or the date when the changing is performed.

In another aspect of the system of the present invention, the database correlates in-charge data, which indicate an organization in charge which takes charge of the patient or a person in charge who takes charge of the patient, with the medical care data and thereby stores the correlated in-charge data, and the search device performs a search on the basis of the in-charge data, by using a condition that the organization or the person in charge is the same.

According to this aspect, the database correlates the in-charge data indicating the unit such as a hospital unit, a hospital ward unit, a hospital sector unit, a department unit, a group of nurses unit, a group of doctors unit etc. which takes charge of the patient, with the medical care item data and stores the correlated in-charge data. Then, the search device performs the search on the basis of the in-charge data by the condition that the organization or the person in charge is the same, so that a list about a group of the patients for whom the organization in charge or the person in charge is the same is displayed. Therefore, if the display device is installed at a place where the organization in charge or the person in charge waits or passes through, they can perform the confirming of the order for the plurality of patients of whom they themselves take charge, effectively and without omission.

In another aspect of the system of the present invention, the search device performs the search with such a search condition that a searching object will be performed during a predetermined period including the present date as a standard day.

According to this aspect, the search device performs the searching with such a search condition that the searching object will be performed during the predetermined period, for example, within 4 days, within 3 days, within 2 days etc. including the present date as the standard day, so that it is possible to display the table regarding the important medical care item or items to be confirmed or to be executed around the present date. Consequently, it is possible for the nurse or the like to perform an operation such as the confirming of the order to be actually confirmed or to be actually executed by the nurse herself at the present time, effectively and without omission.

In another aspect of the system of the present invention, the display device displays the symbol mark within the corresponding cell on the table if the condition data indicate such a condition that an order associated with the medical care item has been issued, and the display device does not display the symbol mark within the corresponding cell on the table if the condition data indicate such a condition that an order associated with the medical care item has been already confirmed.

According to this aspect, the display device displays the symbol mark e.g. the mark showing "○" within the corresponding cell on the table as for the medical care item for which the order has been issued or given, and the display device does not display the symbol mark within the corresponding cell, that is, the display device leaves the cell blank as for the medical care item for which the order has been confirmed. Consequently, the nurse or the like can distinguish the medical care item to be actually confirmed visually and easily because the blank is given to the confirmed medical care item while the symbol mark is given to the medical care item to be confirmed.

In another aspect of the system of the present invention, a symbol mark indicating an urgent identification among the symbol marks is in a visually specific manner as compared with the rest of the symbol marks.

According to this aspect, the symbol mark for the urgent instruction is displayed in a visually specific manner such as red display, blinking display, luminous display etc., so that it is possible to decrease the possibility that the nurse, the doctor or the like overlooks the urgent instruction.

In another aspect of the system of the present invention, the symbol mark in the visually specific manner is displayed within the cell corresponding to the table if there is a predetermined word or words in text-information included in the medical care data.

By constituting the system in this manner, if there is a predetermined word or words in text-information included in the medical care data even if data such as a tag, a label, or a flag indicating the urgent instruction are not given to the medical care data including the medical care item indicating the urgent instruction, the word or words are regarded as the data indicating the urgent instruction and the symbol mark in the visually specific manner is displayed, which is extremely useful in practice.

In another aspect of the system of the present invention, the display device displays a column indicating whether or not a medication for the patient will expire during a designated period in addition to the table, and further displays a symbol mark indicating whether or not the medication will expire within the column.

According to this aspect, the display device displays a column indicating whether the medication for the patient will expire during the designated period above the table or adjacent to the table on the picture plane. Then, the display device displays the symbol mark indicating whether or not the medication will expire during the designated period within the column. For example, the symbol mark showing "○" is displayed within a column indicating an expired prescription or a column indicating an expired injection if the prescription or the injection will expire within 4 days with respect to each patient displayed on the table. Consequently, the doctor, the nurse, or the like can certainly realize the fact that the medication will expire before it occurs by returning toward the past on the picture plane for the designated period from the timing when the medication ends. Moreover, the symbol mark indicating the expired prescription is kept displaying as long as the situation that the medication will expire during the designated period continues, so that it is possible to release anxiety and stress about outbreak of the expired medication from the doctor, the nurse or the like.

In this aspect, the input device may be constructed to designate the designated period.

By constituting the aiding system in this manner, it is possible to set the designated period regarded as the most suitable period to the reality and the policy among the individual concrete reality and the policy of the medical care facility where the nurse, the doctor or the like works or where the aiding system is used, which is useful in practice.

In another aspect of the system of the present invention, the search device changes the search condition in correspondence with an operator of the system, and the display device changes at least one of (i) the patient set on the first axis, (ii) the medical care item set on the second axis, and (iii) the symbol mark displayed within the cell depending upon the operator.

According to this aspect, the patient, the medical care item, or the symbol mark on the table is changed according to the operator. For example, if the operator is the nurse, the following is displayed: it is scheduled; the order has been performed; the execution has been performed; (there is a result); it is urgent; it is general etc. If the operator is the doctor, the following is displayed: it is scheduled; it is timing to be ordered; the order has been executed; (there is a result); there is a new result, it is urgent, it is general etc. Incidentally, the aiding system may be constructed such that the substantial operation is started after the operator ID is inputted in order to identify the operator, for example.

In another aspect of the system of the present invention, the aiding system has a plurality of client apparatuses, each including the input device and the display device and being connected to a server apparatus, which includes the database and the search device, through a communication device.

According to this aspect, it is possible to set the plurality of the client apparatuses separately to the plurality of operators such as the plurality of the nurses and the like who take charge of different patients. It is also possible to set the plurality of the client apparatuses separately to the plurality of operators such as the plurality of the nurse and the like who perform their different duties. This makes it possible for each operator to perform the confirming separately on the basis of the same database. Moreover, the system may be constructed such that the proper medical care item, the proper patient, or the proper symbol mark is displayed, which is appropriate for the operator, who operates each client apparatus.

The above object of the present invention can be achieved by a method for aiding to confirm a medical care item. The method is provided with: a search process of searching a database storing medical care data with a search condition corresponding to a plurality of medical care items and a plurality of patients to be confirmed, the medical care data including (i) medical care item data indicating the medical care item in one condition of a plurality of types of conditions, which are set in advance, (ii) condition data indicating the one condition, and (iii) patient identification data indicating the patient associated with an execution of the medical care item; a first display process of displaying a table in a matrix shape in which the patient is set on a first axis and the medical care item is set on a second axis on the basis of the medical care data which is obtained as a search result by the search process and of displaying one symbol mark assigned to one condition indicated by the condition data included in the medical care data obtained as the search result, among a plurality of symbol marks assigned to a plurality of types of condition marks respectively, within a corresponding cell on the table; a first input process of designating a desired cell or symbol mark, or a desired patient on the displayed table; a second display process of displaying at least one portion of the cell or the symbol mark designated by the input process or the medical care data associated with the patient in a predetermined format different from a format of the table in place of or overlapped with the table, or together with the table; a second input process of changing the condition data indicated by the symbol mark displayed by the first display process; and a third display process of displaying the symbol mark indicating the condition data which is changed within the corresponding cell after the second input process changing the condition data.

According to the aiding method of the present invention, because the aiding method operates in the same manner as the above described the aiding system of the present invention, it is possible for the nurse or the like to take a survey of the conditions of the plurality of the medical care items relating to the plurality of patients by looking at the table having the symbol marks indicating the conditions of the medical care items such as the item scheduled by the doctor or the like and the item for which the order is issued by the doctor or the like within the corresponding cell thereof. This makes it possible to decrease the possibility of occurring a confirmation error and malpractice.

The aiding method of the present invention can be realized in various aspects in correspondence with the above described various aspects of the aiding system of the present invention.

The above object of the present invention can be also achieved by a computer program of instructions executable by a computer to perform the above described aiding method of the present invention (including its various aspects). More concretely, the computer program of instructions causes the computer to perform each process such as the search process, the first and second display process, the first and second input process etc., which constitute the aiding method of the present invention.

According to the computer program of the present invention, the above described aiding system of the present invention can be realized as a computer reads and executes the program of instructions from the program storage device such as a CD-ROM (Compact Disc - Read Only Memory), a DVD-ROM (DVD Read Only Memory), a hard disc or the like, or as it executes the program of instructions after downloading the program through a communication device.

The nature, utility, and further features of this invention will be more clearly apparent from the following detailed description with respect to preferred embodiments of the invention when read in conjunction with the accompanying drawings briefly described below.

### BRIEF DESCRIPTION OF THE DRAWINGS

FIG. 1 is a block diagram of a system for aiding to confirm a medical care item of a first embodiment of the present invention;
FIG. 2 is a schematic diagram showing a schematic data structure of one example of a database used in the first embodiment;
FIG. 3 is a flow chart showing an operation to display a table or the like to confirm the medical care item of the first embodiment;
FIG. 4 is a flow chart showing one concrete example of an operation of generating display data among the operations in the first embodiment;
FIG. 5 is a plan view showing one example of a display which is displayed on one or whole portion of a picture plane of a display device of the first embodiment;
FIG. 6 is a plan view showing another example of a display which is displayed on one or whole portion of the picture plane of the display device of the first embodiment;
FIG. 7 is a plan view showing another example of a display which is displayed on one or whole portion of the picture plane of the display device of the first embodiment;
FIG. 8 is a plan view showing another example of a display which is displayed on one or whole portion of the picture plane of the display device of the first embodiment;
FIG. 9 is a plan view showing another example of a display which is displayed on one or whole portion of a picture plane of the display device of the first embodiment;
FIG. 10 is a plan view showing a table displayed by a system for aiding to confirm a medical care item of a second embodiment of the present invention;
FIG. 11 is a block diagram showing the whole structure of a system for aiding to confirm a medical care item of a third embodiment of the present invention; and
FIG. 12 is a flow chart showing a process of setting a condition during an operation of the third embodiment.

### DETAILED DESCRIPTION OF THE PREFERRED EMBODIMENTS

Referring to the accompanying drawings, embodiments of the present invention will be now explained.

### (I) First Embodiment

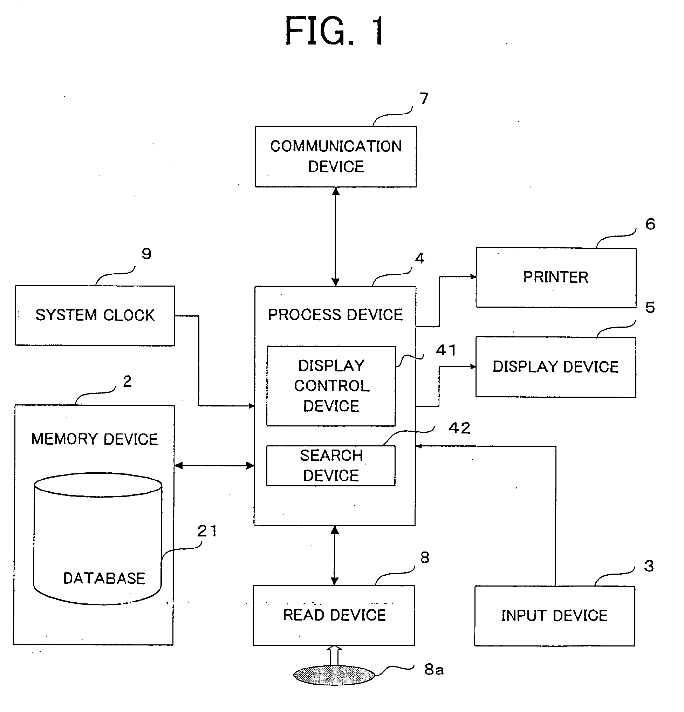

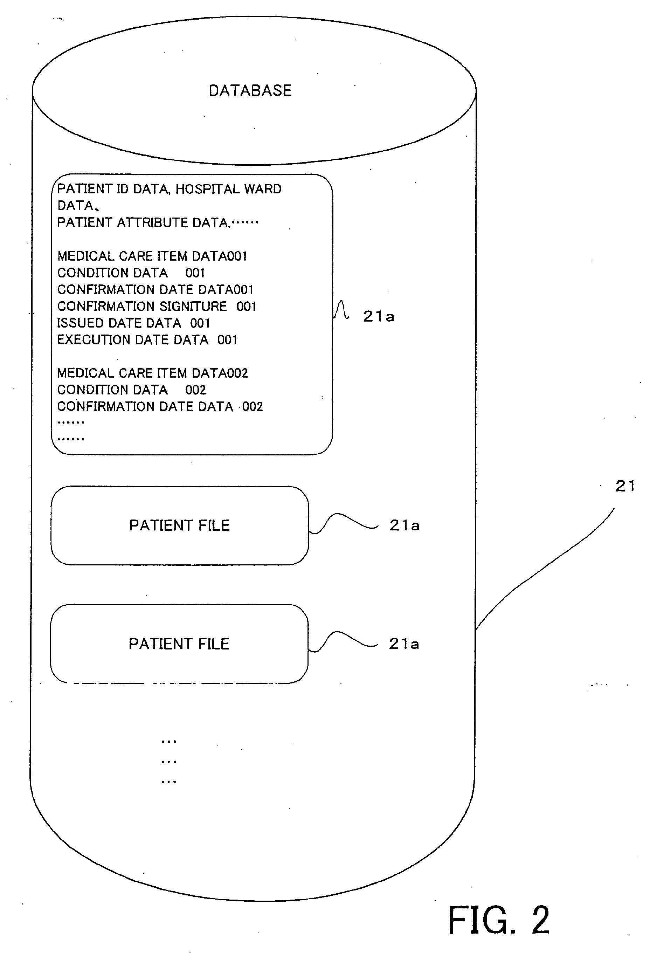

FIG. 1 is a block diagram of a system for aiding to confirm a medical care item of a first embodiment of the present invention. FIG. 2 is a schematic diagram showing a schematic data structure of one example of a database used in the first embodiment.

In FIG. 1, a system for aiding to confirm a medical care item may comprise, as a hardware resource, a personal computer, a work station, a middle size computer, a large size computer, a mobile computer (i.e., a hand-carry type information terminal), an electronic diary or the like. The aiding system is provided with: a memory device 2; an input device 3; a process device 4; a display device 5; a printer 6; a communication device 7; a read device 8; and a system clock 9.

The memory device 2 is preferably a known large data volume memory device of randomly accessible type, such as a hard disc device, an IC (Integrated Circuit) memory, a magnetic disc device, a magneto-optical disc, an optical disc device or the like.

A database 21 is constructed in the memory device 2.

As shown in FIG. 2, the database 21, for example, includes many patient files 21a, each of which is set for respective one of the patients. Each of the patient files 21a includes various types of medical care data such as patient ID data, hospital ward data, patient attribute data etc., which are code-formed, text-formed or the like, for an electric health record with reference to the patient. Moreover, each of the patient files 21a includes medical care item data showing the medical care item which is in one condition (i.e., one status) among a plurality of kinds of conditions (i.e., a plurality of kinds of statuses) set in advance. As the plurality of kinds of conditions, there are for example, the condition that the medical care item is scheduled by a doctor or the like, the condition that an order associated with the medical care item has been already issued or given by a doctor or the like, the condition that the order associated with the medical care item has been already confirmed by a nurse or the like, the condition that the medical care item has been already executed by a nurse or the like. Each of the patient files 21a also includes: condition data, which shows the one condition; confirmation date data, which show the date when a nurse or the like confirms the corresponding medical care item; a confirmation signature (data), which identifies the person such as a nurse or the like who confirms the medical care item; issued date data, which indicates the date when a doctor or the like issues or give an order; execution date data, which indicates the date when a nurse or the like executes the medical care item according to the order; and so on.

In FIG. 1 again, the input device 3 is provided with a key board, a ten key switch, a mouse, a track ball, an input pen, an input tablet, a sound input device or the like, and is adapted to input the medical care item data, the condition data, the confirmation signature as well as other various data or commands, and is further adapted to specify or designate an arbitrary position on the image displayed on the display device 5.

The process device 4 has a CPU (Central Processing Unit), and there are constructed a display control device 41, which controls a display on the display device 5, as described later, and a search device 42, which searches the database 21, as described later. Incidentally, in the present embodiment, there is constituted one example of the display device, which displays a table having a symbol mark for confirming or which displays the content of the medical care items corresponding to the symbol mark in a different format, by using the display control device 41 constructed in the process device like the above described, and by using the display device 5.

The display device 5 may be a known display device such as a CRT (Cathode Ray Tube) display device, an LCD (Liquid Crystal Display) device or the like, and is constructed to display various types of display data under the control by the display control device 41; also, the display device is constructed such that an arbitrary position on its picture plane can be specified or designated by the input device 3.

The printer 6 may be a known printer such as a laser beam printer, an ink jet printer or the like, and may be a color type or a black and white type. The printer 6 is constructed to print an arbitrary picture plane displayed on the display device 5 (e.g., the table having symbol marks for confirming etc.) by inputting a predetermined printing command through the input device 3.

The communication device 7 is provided with a modem etc., to perform a data communication of various files including the object files 21 and data with another computer or the like. The communication device 7 is connected with other large size computer, personal computer, mobile computer (i.e., a hand-carry type information terminal), an electronic diary and the like, through a communication line, such as a wire-line, a wireless-line, an exclusive line, a general line, a telephone line and so on.

The read device 8 may include a CD-ROM drive, a DVD-ROM drive and an FD (Floppy or Flexible Disk) drive for reading a computer program recorded on a record medium 8a, such as a CD-ROM, a DVD-ROM and an FD respectively, for example. The computer program read in this manner allows the computer i.e., the hardware resource of the system to function as the aiding system. Incidentally, it is possible to record one or whole portion of the database constructed in the memory device 2 in a record medium 2a and to read out it as the occasion demands.

The system clock 9 has a calendar function and always measures the present date and time regardless of the on/off of the main power of the aiding system. The process device 4 refers to the date and time measured by the system clock 9 when recognizing the present date. The aiding system may be constructed such that the process device 4 refers to the present date and time signal of a clock device, which is installed at the external of the aiding system and which outputs the present date and time signal indicative of the present date and time at the constant cycle, in place of the system clock 9, to thereby obtain the present date and time.

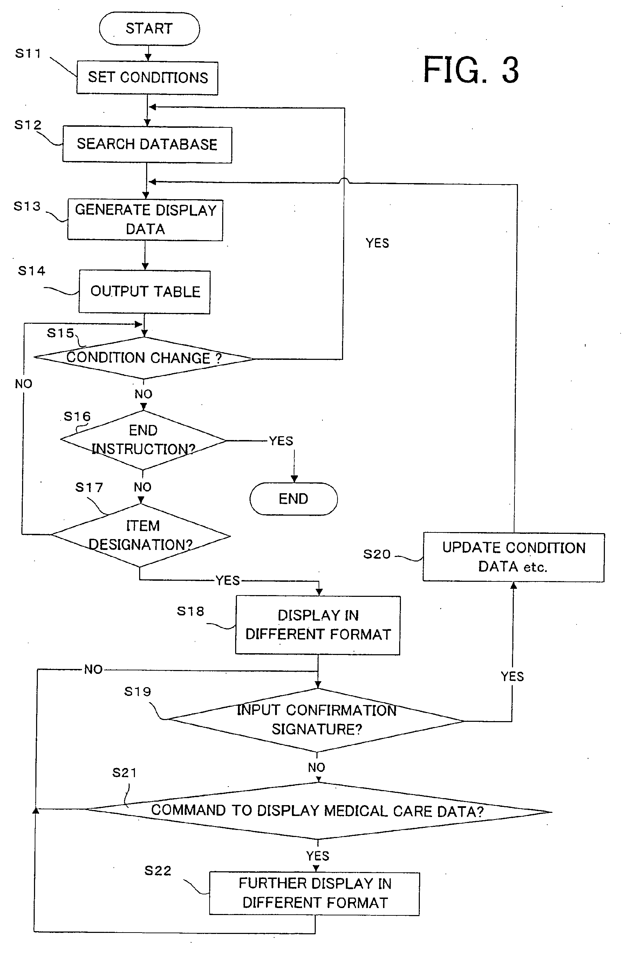

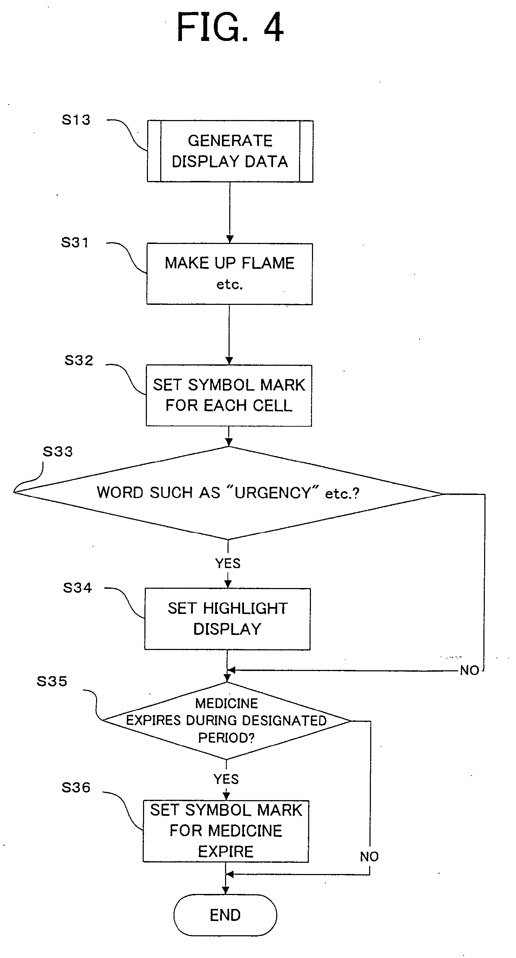

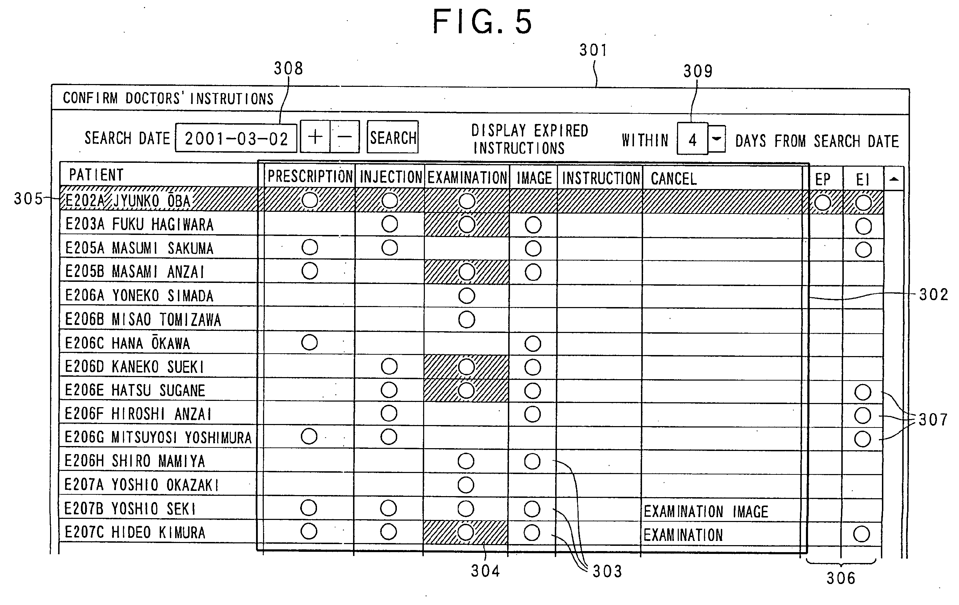

Next, an operation to display the table or the like to confirm the medical care item in the aiding system constructed in the manner as described above will be explained with reference to the flow charts in FIG. 3 and FIG. 4, and with reference to each of display examples in FIG. 5 to FIG. 9. Here, FIG. 3 is a flow chart showing the operation to display the table or the like in order to confirm the medical care item of the first embodiment. FIG. 4 is a flow chart showing one concrete example of an operation of generating display data among the operations of the first embodiment. Each diagram shown from FIG. 5 to FIG. 9 is a plan view showing the example of a display which is displayed on one or whole portion of a picture plane of the display device 5 of the first embodiment.

Firstly, in FIG. 3, the conditions about a plurality of patients and a plurality of the medical care items, which will be included on the table to confirm the medical care item displayed on the display device 5, are set. Here, for example, in order to list up the plurality of patients, whom the same group of nurses or the like handle, on the table without any excess and deficiency, the followings are set as the conditions: a hospital ward, a hospital sector, a hospital department etc., which are handled by the same group of nurses or the like and for each of which the display device 5 is disposed respectively. By which unit the same group of nurses take charge of the patients depends on the practice of the medical care facility. Secondly, the following plurality of medical care items are set as the conditions: a prescription, an injection, an examination, photographing an image, instructions to the patients and the like etc., which are the items to be confirmed and to be executed by those kind of group of nurses (step S11). At this time, such a fact that the order is issued for the medical care item by a doctor or the like or that the medical care item is scheduled during designated days (e.g., 4 days) including the present date as a standard day obtained from the system clock 9, may be chosen as the condition. Incidentally, this kind of the setting of the search condition or conditions can be performed automatically on the basis of where the display device 5 is disposed or on the basis of "the present date". In place of that or in addition to that, the setting of the search condition or conditions may be performed by an operator such as a nurse, a doctor or the like through the input device 3.

Next, according to the search conditions as set in the above manner, the database 21 shown in FIG. 2 is searched (step S12). Here, for example, the hospital ward data indicative of the hospital ward which meets the search conditions or the issued date data which meet the search conditions are used as a search key word, and the searching is performed by a unit of the medical care item data in each patient file 21a. Then, on the basis of the medical care data obtained as a search result, the following display data are generated (step S13): the display data to display the table in a matrix shape, in which a plurality of the patients are set on an ordinate, i.e. a vertical axis and in which a plurality of the medical care items are set on an abscissa, i.e. a horizontal axis; and the display data to display one symbol mark assigned to one condition, which is indicated by the condition data included in the medical care data obtained as the search result, among a plurality of the symbol marks assigned to a plurality of types of conditions, within the corresponding cell on the table. Incidentally, as for the plurality kinds of the symbol marks, (i) a symbol mark showing "○" displayed within a cell, and (ii) a "blank", in which nothing is displayed within a cell, are treated as two types of the marks in order to simplify a situation.

More concretely, for example, the step S13 is as follows, as shown in FIG. 4. Firstly, a basic structure such as a frame or the like of the table is made up by deciding the table's ordinate, i.e. a vertical axis, on the basis of the patient ID obtained as the search result and by deciding the table's abscissa, i.e. a horizontal axis, on the basis of the medical care item obtained as the search result (step S31). Secondly, the symbol mark, which is set in advance for each condition, is set in each cell within an area 302 on a table 301 described later on the basis of the condition data obtained as the search result (refer to FIG. 5) (step S32). Thirdly, it is judged whether or not a certain word such as "urgency", "ASAP", or the like is contained, for example, in text data, which is obtained as the search result and which constitutes the medical care item data associated with the order from a doctor or the like (step S33). If the text data contain the word "urgency" etc., (step S33: YES), a highlight-display is set for the corresponding symbol mark (step S34), and the operational flow proceeds to step S35. If the text data do not contain the word "urgency" etc., (step S33: NO), the operation flow proceeds to the step S35 without anything else performed. Then, it is judged whether or not a medication expires, i.e. whether or not a prescription expires or whether or not an injection expires, during the designated period (step S35). If the medication expires (step S35: YES), the symbol mark, which is determined in advance for the expired medication, is set in the corresponding cell within an area 306 on the table 301, as described later (refer to FIG. 5) (step S36), and the generation of the display data is ended. If the medication does not expire (step S35: NO), it is ended to generate the display data without anything else performed.

In FIG. 3 again, the display device 5 outputs and displays the display data generated in the step S13 in the manner as described above on one or whole portion of the picture plane of the display device 5, as the table 301 to confirm the medical care item which is shown in FIG. 5 for example, under the control by the display control device 41 (step S14).

Here, each of the symbol marks 303 of "○" is displayed within the respective one of the cells corresponding to the medical care item to which a doctor or the like has given the order and for which a nurse or the like has not confirmed the order yet, with respect to each patient and each medical care item within the area 302 enclosed with a thick line in FIG. 5. On the other hand, the symbol mark 303 of "○" is not displayed within the respective one of the cells corresponding to the medical care item for which a nurse or the like has already confirmed the order, and the cell is kept blank. In place of leaving the cell blank, it is possible to display the symbol-mark in a different shape, in a different color, or in a different display pattern. In addition to the above mentioned display according to whether or not the order has been confirmed, it is possible to show the conditions such as the condition that the schedule is being made but the order is not issued yet, the condition that the order has been confirmed and further executed, and so on, with the symbol marks indicating the conditions according to the corresponding condition data.

In FIG. 5, each of the symbol marks 304 of highlight-displayed "●" is displayed for the respective one of the medical care items corresponding to urgent instructions. In place of the highlight-display in this kind, it is possible to display the symbol mark 304 in a different color such as red, orange etc. and to display the mark in a different shape such as 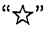 , "!" etc., in order to get attention.

On the other hand, in FIG. 5, each of the symbol marks 307 of "○" is displayed within the respective one of the corresponding cells in the case that a prescription instruction will expire within the designated period (in this case, within 4 days) shown in a designated period column 309 within the area 306 adjacent to the right-hand side of the area 302 enclosed with the thick line, with reference to each patient. Here, the prescription is the one to which a doctor or the like has given the order, and the designated period is decided on the basis of the search date (in this case, March 2, 2001) shown in the search date column 308. In the same manner, each of the symbol marks 307 of "○" is displayed within the respective one of the corresponding cells in the case that an injection instruction will expire within the designated period with reference to each patient. Here, the injection is the one to which the doctor or the like has given the order, and the instruction period is decided on the basis of the search date.

It is possible to change the date data and the designated period data, which are respectively displayed in the search date column 308 and the designated period column 309 displayed within the table 301, as mentioned above, by using the input device 3. As a default value, the present date is used as the search date according to the system clock 9, and it is less necessary to change the designated period often.

Moreover, a line 305 for the patient located in the top line is inversion-displayed on the table 301 shown in FIG. 5. This shows that the line 305 is in a chosen condition by designating it through the input device 3.

In FIG. 3 again, it is judged whether there exists any condition change as for the patient and the item constituting the table 301 to be displayed (step S15). More concretely, it is judged whether or not the search condition has been changed through the input device 3 by the nurse, the doctor etc. who desires to display the table 301 having a different group of patients and a different group of the items listed thereon, for example. Then, if there is such a condition change (step S15: YES), the operational flow returns to the step S12, and the searching of the database (step S12), the generating of the display data (step S13), and the outputting of the table (step S14) are repeated.

On the other hand, if there is no condition change (step S15: NO), it is judged whether or not an instruction indicative of ending the display on the table 301 has been performed through the input device 3 (step S16). Here, if this instruction has been performed (step S16: YES), a series of the display process is ended. If this instruction has not been performed (step S16: NO), it is judged whether or not the designation of the item to confirm the order or to confirm the details of the display content has been performed by a nurse or the like, with respect to the table 301 (step S17). If the designation has not been performed (step S17: NO), the operational flow returns to the step S15, and the input operation or the like associated with the step 15 to the step 17 will be continued to be observed under such a condition that the same table 301 is displayed.

Then, if the designation of the item has been performed in the step S17 (step 17: YES), the operational flow diverges to a step S18. Here, it is assumed that the item relating to the line 305 is chosen as shown in FIG. 5, for example.

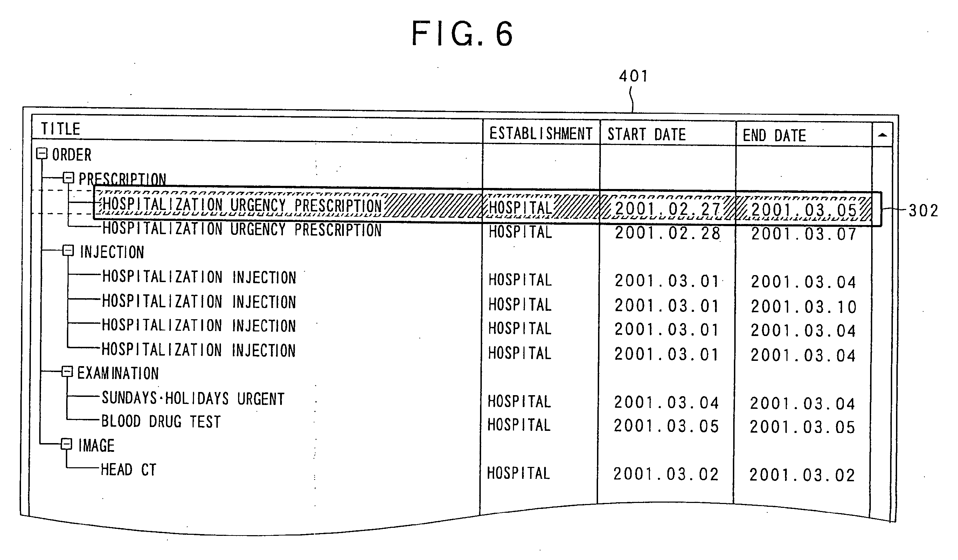

In the step S18, the display device 5 displays the medical care data relating to the item chosen in the step S17 in a different format from the one of the table 301 shown in FIG. 5 under the control by the display control device 41. Here, a window 401 containing the details of the medical care item is displayed, as shown in FIG. 6, for example. Within the table 301 shown in FIG. 5, it is possible to visually recognize the information indicating whether or not the order has been roughly confirmed by the presence or absence of the symbol mark 303. On the other hand, on the window 401 shown in FIG. 6, it is possible to recognize the detailed content of each medical care item. Furthermore, the window 401 may be constructed such that the order which has been confirmed by a nurse or the like is text-displayed in black and the order which has not been confirmed yet by a nurse or the like is text-displayed in red, so that it is possible to visually recognize that the order has not been confirmed yet without the symbol mark 303 therein.

Incidentally, a line 402 for the medical care item located in the top line is inversion-displayed on the window 401 shown in FIG. 6. This shows that the line 402 is in a chosen condition by the designating through the input device 3.

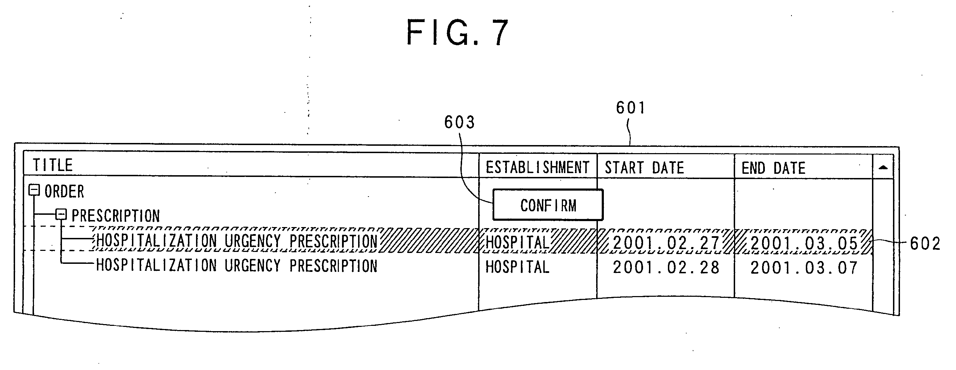

In FIG. 3 again, under the condition that the window 401 is displayed in this manner as described above, it is monitored whether or not inputting the confirmation signature (data) is performed through the input device 3 (step S19). The inputting of the confirmation signature (data) corresponds to an operation to confirm the medical care item data for which the order has not been confirmed yet. For example, the inputting operation may be performed as follows: a confirmation button 603 appeared within a window 601, as shown in FIG. 7, is displayed according to an operation such as mouse-clicking or the like at the input device 3 while the line 402 shown in FIG. 6 is being chosen, then, the inputting of the confirmation signature (data) is performed by operating the confirmation button 603 which is controlled by an further operation such as mouse-clicking or the like at the input device 3. Incidentally, in FIG. 7, the line 602 which is being chosen is also inversion-displayed, so that it is possible to easily understand for which medical care item the confirmation signature is being performed.

In FIG. 3 again, if there exists the inputting of the confirmation signature (step S19: YES), the condition data relating to the corresponding medical care item is updated from the condition data indicating the condition that the order has been issued or given, to the condition data indicating the condition that the order has been confirmed (step S20), and then the operational flow returns to the step S13 to repeat the steps thereafter.

Incidentally, when the updating in the step S20 in this kind is performed, operator identification data indicating an operator, e.g. a certain nurse or the like, who updates the condition data, and change date data indicating the date when the changing is executed are also updated within the patient file 21a in a form corresponding to the condition data. This makes it possible to specify or confirm the operator and the date which will be associated with the updating later. Moreover, it may be constructed such that at least one of the operator identification data and the change date data is displayed in place of or in addition to the table 301 shown in FIG. 5 by the display device 5.

On the other hand, if there does not exist the inputting of the confirmation signature (step S19: NO), it is judged whether or not an order to display the medical care data relating to the medical care item shown in FIG. 6 is performed through the input device 3 (step S21). Here, if the order to display the medical care data is not performed (step S21: NO), the operational flow returns to the step S19 to continue observing in the step 19 and the step 20, while the display like FIG. 6 which is displayed in the step S18 is being continued.

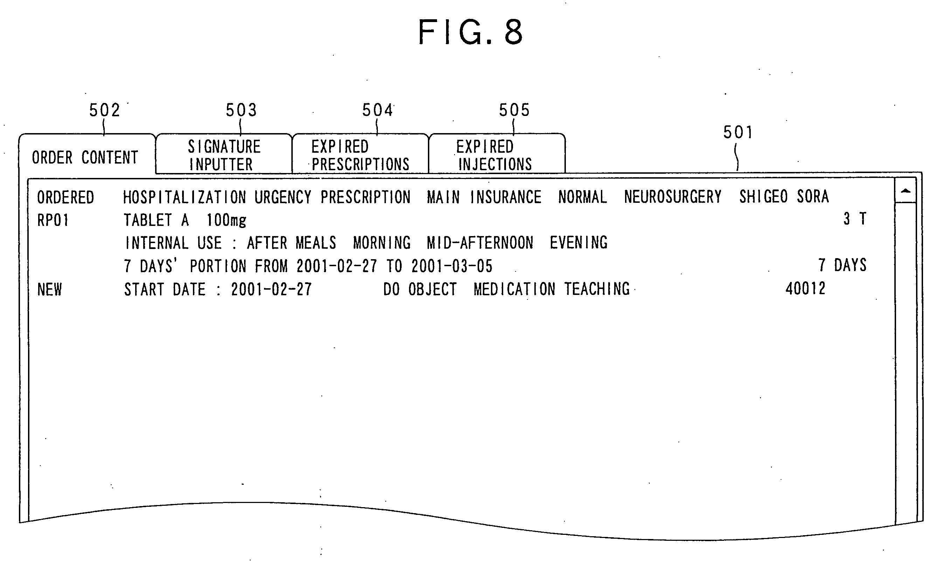

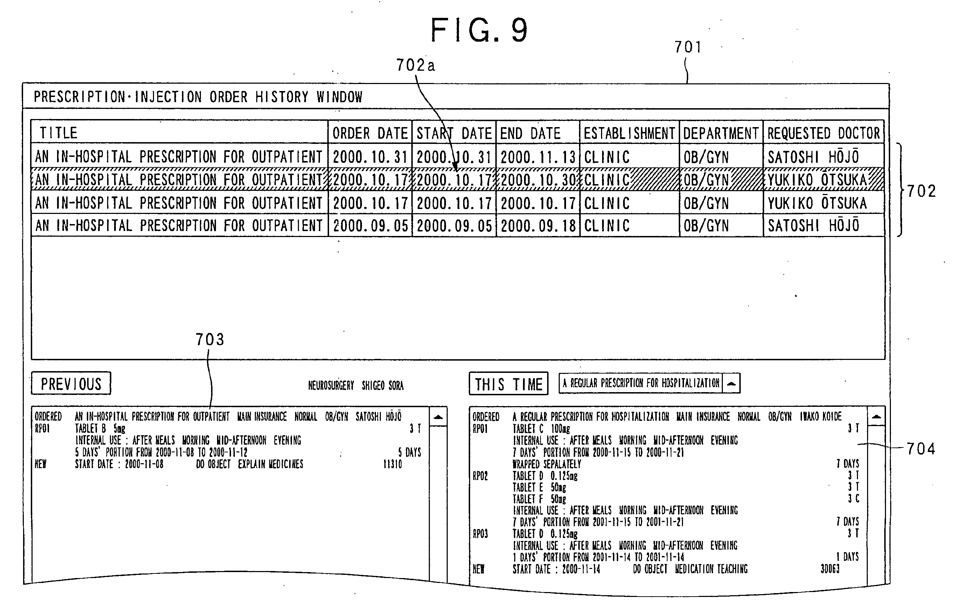

On the other hand, in the step S21, if the order to display the medical care data is performed (step S21: YES), the display device 5 further performs the display in a different format under the control of the display control device 41 (step S22). Here, for example, like a window 501 shown in FIG. 8, the display device 5 displays a window 502 showing an order content, a window 503 showing one that inputs signature, a window 504 showing that the prescription instruction is expired, or a window 505 showing that the injection instruction is expired with reference to the medical care item 402 which is being chosen. Alternatively, like a window 701 shown in FIG. 9, the display device 5 displays a list 702 containing the prescription associated with the line 602 shown in FIG. 7. In FIG. 9, the window 701 contains therein an area 703 displaying the content of the order which was issued previous time and an area 704 displaying the content of the order which is performed this time, wherein both order are with respect to the prescription associated with a chosen line 702a. This is extremely useful in practice because it is possible to decide the content of the order performed this time with reference to the content of the order performed the previous time. The window 701 shown in FIG. 9 may be also constructed such that the window 701 is displayed by clicking and designating the symbol mark 307 displayed in the area 306 within the table 301 shown in FIG. 5.

In FIG. 3 again, after the step S22 is performed, the operational flow returns to the step S19 to continue the display like FIG. 8 or FIG. 9 displayed in the step S21 and to continue monitoring in the step S19 to the step S22.

As described above, according to the system of the first embodiment, it is possible for the nurse or the like to take a survey of the whole condition of the plurality of the medical care items relating to the plurality of patients whom the nurse herself or the group to which she belongs handle, by looking at the table 301 etc. having the symbol marks 303 etc. indicating the condition of the medical care items such as the item scheduled by the doctor or the like and the item for which the order is issued by the doctor or the like within the corresponding cell thereof. This makes it possible to decrease the possibility of occurring a confirmation error and malpractice. Especially, the nurse or the like can change the condition data corresponding to the medical care data associated with the symbol mark 303 or associated with the patient on the table 301 etc. through the input device 3 by specifying the symbol mark 303 or the patient, so that the nurse or the like can surely perform the confirmation about each medical care item with the aid of the table 301 etc.

Moreover, according to the first embodiment, because the symbol mark 307, which shows whether or not the medication will expire during the designated period, is displayed in the area 306, as shown in FIG. 5, the doctor, the nurse, or the like can certainly realize the fact that the medication will expire before it actually occurs by the designated period. Therefore, it is possible to release anxiety and stress about the outbreak of the expired medication from the doctor, the nurse or the like.

In the above described embodiment, it often happens that there are many medical care items corresponding to one symbol mark 303 shown in FIG. 5, which is one characteristic of the medical care, as shown in FIG. 6. In this case, the following rule may be adopted: the symbol mark 303 on the table 301 shown in FIG. 5 is deleted after the order is confirmed, with reference to the whole of the plurality of the medical care items corresponding to the one symbol mark 303. In the embodiment constructed in the above mentioned manner, it is possible to decrease the possibility to occur an error such as a partial lack of the confirmation if the confirmation is carefully performed especially about the cell having the symbol mark 303. The embodiment may be also constructed such that the display form of the symbol mark 303 is changed according to the number and the ratio of the medical care item or items, for which the order is confirmed, of the plurality of the medical care items corresponding to one symbol mark 303.

The above described embodiment may be constructed such that an examination result data are displayed by designating the medical care item on the windows with the input device 3 etc. as for the medical care item which relates to the medical care data displayed on the various types of windows shown in FIG. 5 to FIG. 9 and which is accompanied by the examination result, such as an executed general examination and an executed image examination.. Moreover, in the above described first embodiment as well as each embodiment described below, it is possible to display "record by doctor or nurse", "process", "injection, "examination", "test", "evaluation", "medication", "meal (food)", "practice", "monitor", "treatment", "activity restriction", "observation", "rehabilitation", "coordination", "hospitalization and leave of hospital", "education for family of patient" and so on, as the medical care item in addition to " prescription" and "injection" shown in FIG. 5 to FIG. 9. Moreover, each embodiment may be constructed such that these medical care items are displayed in the way of a hierarchy category system comprising large categories and small categories belonging to each large category.

Furthermore, in the above described first embodiment, each of various types of the windows shown in FIG. 5 to FIG. 9 may be displayed on the whole picture plane of the display device 5 by switching an image. It may also be displayed such that two or more windows are adjacent to each other horizontally or vertically, or such that two or more windows are overlapped, by separate-displaying or window-displaying or the like. It may be further scroll-displayed by a unit of the window which is separate-displayed or window-displayed.

### (II) Second Embodiment

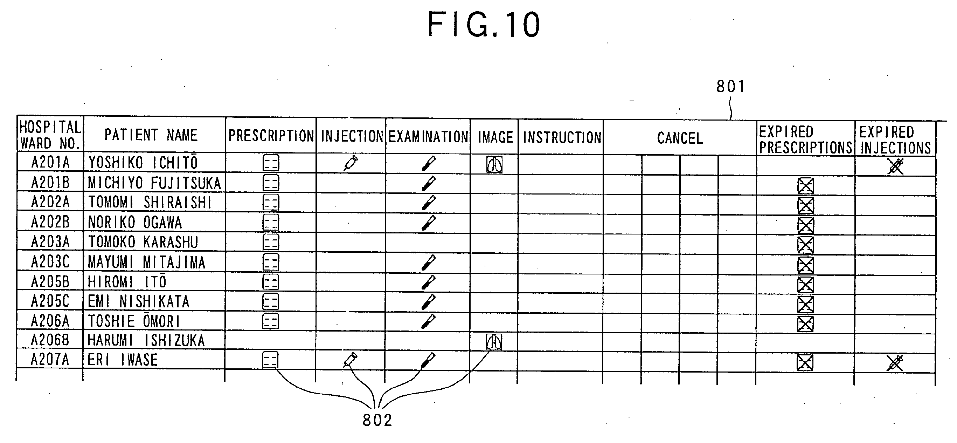

A system for aiding to confirm a medical care item of a second embodiment of the present invention will be explained with reference to FIG. 10. FIG. 10 is a plan view showing a table displayed by the system of the second embodiment of the present invention.

The system of the second embodiment is constructed such that the hardware structure thereof is the same as that of the first embodiment described above and such that the operation to display the table to confirm the medical care item is the same as that of the first embodiment, except that the type of symbol mark thereof is different.

As shown in FIG. 10, in the second embodiment, the display device 5 displays a table 801 to confirm the medical care item under the control of the display control device 41. Especially, a symbol mark 802 including various types of picture patterns is displayed within each cell on the table 801.

Therefore, according to the second embodiment, looking at the table 801 makes it possible to identify the medical care item indicating the order confirmation to be performed more visually and more easily.

### (III) Third Embodiment

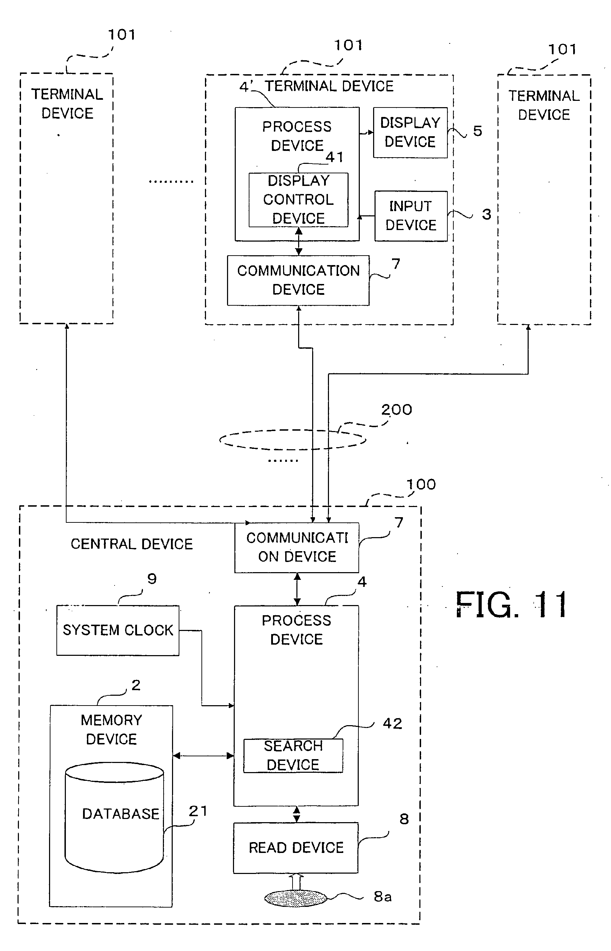

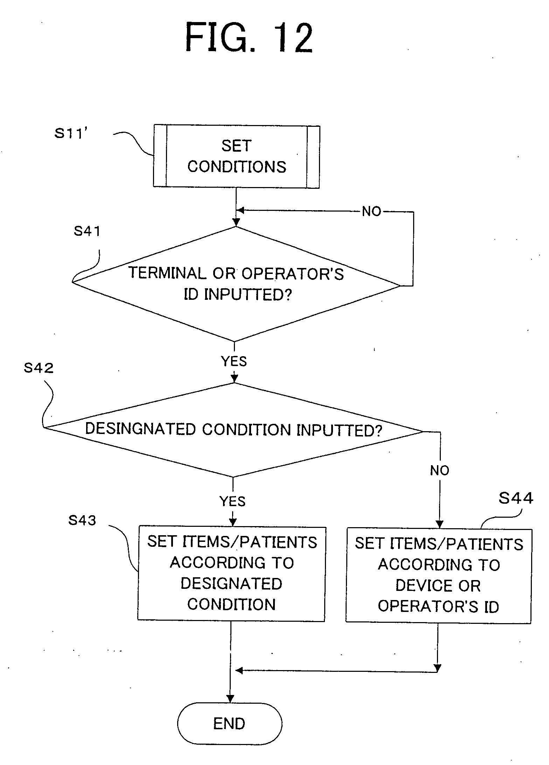

The third embodiment of the present invention will be explained with reference to FIG. 11 and FIG. 12. FIG. 11 is a block diagram showing the whole structure of a system of the third embodiment. FIG. 12 is a flow chart showing an operation of setting a condition during the operations of the third embodiment.

In FIG. 11, the system for aiding to confirm the medical care item of the third embodiment is provided with a plurality of units communicated through a communication line 200, which may be a wire-line, a wireless-line, an exclusive line, a general line, a telephone line or the like. A central device 100 is provided with: the memory device 2 for storing the database 21; the communication device 7; the read device 8; and the system clock 9. On the other hand, each of a plurality of terminal devices 101 is provided at least: the input device 3; the process device 4' equipped with the display control device 41; the display device 5; and the communication device 7. A terminal device 101 comprises, for example, a personal computer, a work station, a mobile computer (i.e., a hand carry type information terminal), an electronic diary or the like.

Therefore, by virtue of such a structure that the database 21 are stored in the large size memory device 2 equipped on the central device 100 and that a plurality of terminal devices 101 are arranged, it is possible to use the same data commonly by a plurality of terminal devices 101. This is practically advantageous because there is no need to have a large size memory device 2 for storing the database 21 in each terminal device 101. Moreover, the third embodiment may be constructed such that the terminal device 101 is usually provided only with a browser function and such that the table etc. to confirm the medical care item shown in FIG. 5 to FIG. 9 is displayed on the side of the terminal device 101 according to the data and the program provided from the central device 100.

In the third embodiment, moreover, it is possible to set a plurality of the terminal device 101 separately to a plurality of operators such as the plurality of the nurse and the like who take charge of different patients. It is also possible to set the plurality of the terminal device 101 separately to the plurality of operators such as the plurality of the nurse and the like who perform their different duties. In this case, the embodiment may be constructed such that the proper medical care item, the proper patient, or the proper symbol mark is displayed according to the operator who operates each terminal device 101.

In the third embodiment, it is possible to give a small change to the process associated with the condition setting (step S11) shown in FIG. 4, so that the third embodiment may be performed as follows.

Namely, in place of the condition setting process (step S11) shown in FIG. 4, as shown in a condition setting process (step S11') shown in FIG. 12, inputting of the terminal or the operator's ID is firstly monitored (step S41). If the inputting is performed (step S41: YES), it is judged whether or not the inputting of the designated condition is performed arbitrarily (step S42).

If the inputting of the designated condition is performed (step S42: YES), the medical care item and/or the patient in response to the designated condition are set as the search condition (step S43), and the condition setting process is ended (step S11'). On the other hand, if the inputting of the designated condition is not performed (step S42: NO), the medical care item and/or the patient in response to the terminal device 101 or the operator ID of the nurse or the like are set as the search condition (step S44), and the condition setting process is ended (step S11').

In the third embodiment, according to the search condition set in this step S44, it is preferably constructed to generate the display data to display the symbol mark indicating the following if the operator is the nurse: it is scheduled; the order has been performed; the execution has been performed; (there is a result); it is urgent; it is general; and so forth. It is also preferably constructed to generate the display data to display the symbol mark indicating the following if the operator is the doctor: it is scheduled; it is the timing to be ordered; the order has been executed; (there is a result); a new result; and so forth. The embodiment may be constructed such that the reference and the input operation is started after the operator ID is inputted in order to identify the operator, for example.

In addition, the present invention can be applied to not only a medical care in a hospital but also a medical care in house or home as well as medical care attendance or nursing.

## Claims

1. A system (1) for aiding to confirm a medical care item **characterized in that** said system comprises:
a search device (42) for searching a database (21) storing medical care data with a search condition corresponding to a plurality of medical care items and a plurality of patients to be confirmed, the medical care data including (i) medical care item data indicating the medical care item in one condition of a plurality of types of conditions, which are set in advance, (ii) condition data indicating the one condition, and (iii) patient identification data indicating the patient associated with an execution of the medical care item;
a display device (41, 5) for displaying a table in a matrix shape in which the patient is set on a first axis and the medical care item is set on a second axis on the basis of the medical care data which is obtained as a search result by said search device and for displaying one symbol mark assigned to one condition indicated by the condition data included in the medical care data obtained as the search result, among a plurality of symbol marks assigned to a plurality of types of condition marks respectively, within a corresponding cell on the table; and
an input device (3) for designating a desired cell or symbol mark, or a desired patient on the displayed table,
said display device displaying at least one portion of the cell or the symbol mark designated by said input device or the medical care data associated with the patient in a predetermined format different from a format of the table in place of or overlapped with the table, or together with the table,
said input device being constructed to change the condition data indicated by the symbol mark displayed by said display device,
said display device displaying the symbol mark indicating the condition data which is changed within the corresponding cell after said input device changing the condition data.

2. A system (1) according to claim 1, **characterized in that** said input device (3) is constructed to change the condition data corresponding to the medical care data displayed in the predetermined format by said display device.

3. A system (1) according to claim 2, **characterized in that**
the medical care item is subdivided into detailed items, and the condition data corresponding to one cell within the table comprises a plurality of detailed condition data indicating a condition of each one of the detailed items, and
said display device (41, 5) displays at least one portion of the medical care data for each of the detailed items as the predetermined format, and
said input device (3) is constructed to change the detailed condition data corresponding to the medical care data displayed in the predetermined format by said display device, and to change the condition data including the detailed condition data by changing the detailed condition data.

4. A system (1) according to claim 3, **characterized in that**
said input device (3) is constructed to designate a desired portion from among the medical care data displayed for each of the detailed items in the predetermined format, and
said display device (41, 5) displays the medical care data associated with the desired portion designated by said input device, in another format.

5. A system (1) according to any one of claims 1 to 4, **characterized in that** the plurality of conditions include at least one of a condition that the medical care item is being scheduled for execution, a condition that an order associated with the medical care item has been issued or given, a condition that the order associated with the medical dare item has been confirmed, and a condition that the medical care item has been executed.

6. A system (1) according to any one of claims 1 to 5, **characterized in that** said display device (41, 5) displays the medical care data on the basis of the condition data in a different mode in correspondence with the condition thereof as the predetermined format.

7. A system (1) according to any one of claims 1 to 6, **characterized in that** said display device (41, 5) displays an examination result in another format in correspondence with the designation by said input device (3) as for the medical care item which is displayed in the predetermined format and is accompanied with the examination result.

8. A system (1) according to any one of claims 1 to 7, **characterized in that**
the database (21) updates a content stored therein in correspondence with a change of the condition data by said input device (3), and
the database stores at least one of (i) operator identification data identifying an operator who executes the change of the condition data by said input device and (ii) change date data indicating a date when the change of the condition data by said input device is executed, with correlating the one with the condition data.

9. A system (1) according to claim 8, **characterized in that** said display device (41, 5) displays at least one of the operator identification data and the change date data in addition to or in place of the table.

10. A system (1) according to any one of claims 1 to 9, **characterized in that**
the database (21) correlates in-charge data, which indicate an organization in charge which takes charge of the patient or a person in charge who takes charge of the patient, with the medical care data and thereby stores the correlated in-charge data, and
said search device (42) performs a search on the basis of the in-charge data, by using a condition that the organization or the person in charge is the same.

11. A system (1) according to any one of claims 1 to 10, **characterized in that** said search device (42) performs the search with such a search condition that a searching object will be performed during a predetermined period including the present date as a standard day.

12. A system (1) according to any one of claims 1 to 11, **characterized in that**
said display device (41, 5) displays the symbol mark within the corresponding cell on the table if the condition data indicate such a condition that an order associated with the medical care item has been issued, and
said display device does not display the symbol mark within the corresponding cell on the table if the condition data indicate such a condition that an order associated with the medical care item has been already confirmed.

13. A system (1) according to any one of claims 1 to 12, **characterized in that** a symbol mark indicating an urgent identification among the symbol marks is in a visually specific manner as compared with the rest of the symbol marks.

14. A system (1) according to claim 13, **characterized in that** the symbol mark in the visually specific manner is displayed within the cell corresponding to the table if there is a predetermined word or words in text-information included in the medical care data.

15. A system (1) according to any one of claims 1 to 14, **characterized in that** said display device (41, 5) displays a column indicating whether or not a medication for the patient will expire during a designated period in addition to the table, and further displays a symbol mark indicating whether or not the medication will expire within the column.

16. A system (1) according to claim 15, **characterized in that** said input device (3) is constructed to designate the designated period.

17. A system (1) according to any one of claims 1 to 16, **characterized in that**
said search device (42) changes the search condition in correspondence with an operator of the system, and
said display device (41, 5) changes at least one of (i) the patient set on the first axis, (ii) the medical care item set on the second axis, and (iii) the symbol mark displayed within the cell depending upon the operator.

18. A system (1) according to any one of claims 1 to 17, **characterized in that** said system comprises a plurality of client apparatuses, each including said input device (3) and said display device (41, 5) and being connected to a server apparatus, which includes the database (21) and said search device (42), through a communication device (7).

19. A method of aiding to confirm a medical care item **characterized in that** said method comprises:
a search process of searching a database (21) storing medical care data with a search condition corresponding to a plurality of medical care items and a plurality of patients to be confirmed, the medical care data including (i) medical care item data indicating the medical care item in one condition of a plurality of types of conditions, which are set in advance, (ii) condition data indicating the one condition, and (iii) patient identification data indicating the patient associated with an execution of the medical care item;
a first display process of displaying a table in a matrix shape in which the patient is set on a first axis and the medical care item is set on a second axis on the basis of the medical care data which is obtained as a search result by said search process and of displaying one symbol mark assigned to one condition indicated by the condition data included in the medical care data obtained as the search result, among a plurality of symbol marks assigned to a plurality of types of condition marks respectively, within a corresponding cell on the table;
a first input process of designating a desired cell or symbol mark, or a desired patient on the displayed table;
a second display process of displaying at least one portion of the cell or the symbol mark designated by said input process or the medical care data associated with the patient in a predetermined format different from a format of the table in place of or overlapped with the table, or together with the table;
a second input process of changing the condition data indicated by the symbol mark displayed by said first display process; and
a third display process of displaying the symbol mark indicating the condition data which is changed within the corresponding cell after said second input process changing the condition data.

20. A computer program of instructions executable by a computer to perform method processes for aiding to confirm a medical care item,
**characterized in that** said method processes comprise:
a search process of searching a database storing medical care data with a search condition corresponding to a plurality of medical care items and a plurality of patients to be confirmed, the medical care data including (i) medical care item data indicating the medical care item in one condition of a plurality of types of conditions, which are set in advance, (ii) condition data indicating the one condition, and (iii) patient identification data indicating the patient associated with an execution of the medical care item;
a first display process of displaying a table in a matrix shape in which the patient is set on a first axis and the medical care item is set on a second axis on the basis of the medical care data which is obtained as a search result by said search process and of displaying one symbol mark assigned to one condition indicated by the condition data included in the medical care data obtained as the search result, among a plurality of symbol marks assigned to a plurality of types of condition marks respectively, within a corresponding cell on the table;
a first input process of designating a desired cell or symbol mark, or a desired patient on the displayed table;
a second display process of displaying at least one portion of the cell or the symbol mark designated by said input process or the medical care data associated with the patient in a predetermined format different from a format of the table in place of or overlapped with the table, or together with the table;
a second input process of changing the condition data indicated by the symbol mark displayed by said first display process; and
a third display process of displaying the symbol mark indicating the condition data which is changed within the corresponding cell after said second input process changing the condition data.
